(19) Europäisches Patentamt
European Patent Office
Office européen des brevets

(11) Veröffentlichungsnummer: **0 143 302**
**A2**

(12) # EUROPÄISCHE PATENTANMELDUNG

(21) Anmeldenummer: **84112432.4**

(22) Anmeldetag: **16.10.84**

(51) Int. Cl.⁴: **C 07 C 127/22**, C 07 C 149/273, C 07 C 148/00, A 01 N 37/26

(30) Priorität: **27.10.83 JP 200092/83**

(43) Veröffentlichungstag der Anmeldung: **05.06.85 Patentblatt 85/23**

(84) Benannte Vertragsstaaten: **BE CH DE FR GB IT LI NL**

(71) Anmelder: **NIHON TOKUSHU NOYAKU SEIZO K.K., No.4, 2-chome, Nihonbashi Honcho Chuo-ku, Tokyo 103 (JP)**

(72) Erfinder: **Toyohiko, Kume, 6-7-8, Asahigaoka, Hino-shi Tokyo (JP)**
Erfinder: **Akihiko, Yanagi, 39-15, Namiki-cho, Hachioji-shi Tokyo (JP)**
Erfinder: **Shinichi, Tsuboi, 3-26-1, Hirayama, Apt.3-101, Hino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

(54) **Neue Benzoylharnstoff-Derivate und Zwischenprodukte.**

(57) Die vorliegende Erfindung betrifft neue Benzoylharnstoff-Derivate der nachstehenden Formel (I),

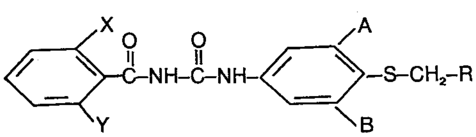
(I),

in der

X und Y jeweils ein Wasserstoff- oder Halogen-Atom bezeichnen, mit der Maßgabe, daß X und Y nicht gleichzeitig ein Wasserstoff-Atom bezeichnen,

A und B jeweils ein Wasserstoff-Atom, eine niedere Alkyl-Gruppe oder ein Halogen-Atom bezeichnen, mit der Maßgabe, daß A und B nicht gleichzeitig ein Wasserstoff-Atom bezeichnen, und

R eine fluoro-substituierte Alkyl-Gruppe mit 1 bis 2 Kohlenstoff-Atomen bezeichnet,

die als Insektizide eingesetzt werden können, und neue Zwischenprodukte zu ihrer Herstellung.

EP 0 143 302 A2

0143302

NIHON TOKUSHU NOYAKU SEIZO K.K., Tokyo, Japan

IVb / ZP

# Neue Benzoylharnstoff-Derivate und Zwischenprodukte

Die vorliegende Erfindung betrifft neue Benzoylharnstoff-Derivate, Zwischenprodukte für deren Herstellung, Verfahren zu ihrer Herstellung sowie Insektizide.

Insbesondere betrifft die vorliegende Erfindung neue Benzoylharnstoff-Derivate der nachstehenden Formel (I).

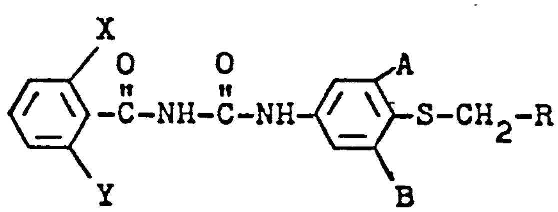

(I)

In der Formel bezeichnen

X und Y jeweils ein Wasserstoff- oder Halogen-Atom, mit der Maßgabe, daß X und Y nicht gleichzeitig ein Wasserstoff-Atom bezeichnen,

A und B jeweils ein Wasserstoff-Atom, eine niedere Alkyl-Gruppe oder ein Halogen-Atom, mit der Maßgabe, daß A und B nicht gleichzeitig ein Wasserstoff-Atom bezeichnen, und

R eine fluoro-substituierte Alkyl-Gruppe mit 1 bis 2 Kohlenstoff-Atomen.

Die neuen Benzoylharnstoff-Derivate der allgemeinen Formel (I) gemäß der vorliegenden Erfindung können mittels der folgenden Verfahren i) und ii) hergestellt werden, auf die sich die vorliegende Erfindung ebenfalls erstreckt.

Verfahren i)

Ein Verfahren zur Herstellung der neuen Benzoylharnstoff-Derivate der allgemeinen Formel (I) umfaßt die Umsetzung einer Verbindung der allgemeinen Formel

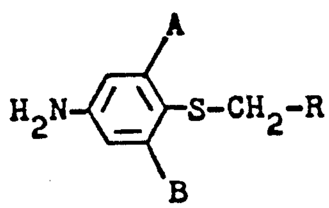

(II)

,

in der A, B und R die im Vorstehenden angegebene Bedeutung haben,
mit einer Verbindung der allgemeinen Formel

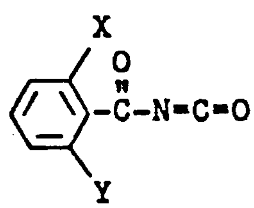

(III)

,

in der X und Y die im Vorstehenden angegebene Bedeutung haben.

Verfahren ii)

Ein Verfahren zur Herstellung der neuen Benzoylharnstoff-Derivate der allgemeinen Formel (I) umfaßt die Umsetzung einer Verbindung der allgemeinen Formel

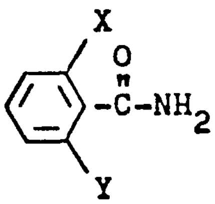

(IV)

,

in der X und Y die im Vorstehenden angegebene Bedeutung haben,
mit einer Verbindung der allgemeinen Formel

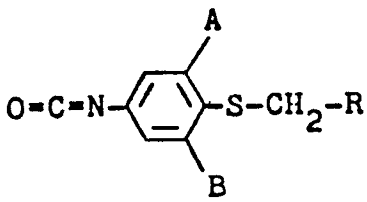

(V)

,

in der A, B und R die im Vorstehenden angegebene Bedeutung haben.

Die vorliegende Erfindung betrifft außerdem Insektizide, die die neuen Benzoylharnstoff-Derivate der allgemeinen Formel (I) als Wirkstoff enthalten.

Bei der Herstellung der neuen Benzoylharnstoff-Derivate der allgemeinen Formel (I) gemäß der vorliegenden Erfindung sind die Anilin-Derivate der allgemeinen Formel (II), die Zwischenprodukte sind, neue Verbindungen, auf die sich die vorliegende Erfindung ebenfalls erstreckt.

Die neuen Anilin-Derivate der allgemeinen Formel (II) können mittels des folgenden Verfahrens hergestellt werden, auf das sich die vorliegende Erfindung ebenfalls erstreckt.

Verfahren iii)

Ein Verfahren zur Herstellung der neuen Anilin-Derivate der allgemeinen Formel (II) umfaßt die Umsetzung einer Verbindung der allgemeinen Formel

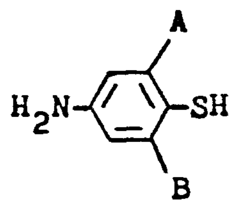

(VI) ,

in der A und B die im Vorstehenden angegebene Bedeutung haben,
mit einer Verbindung der allgemeinen Formel

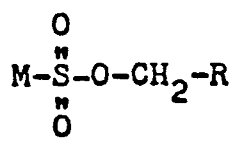

(VII) ,

in der R die im Vorstehenden angegebene Bedeutung hat und M eine niedere Alkyl-Gruppe oder eine Aryl-Gruppe bezeichnet.

Die vor der Einreichung der vorliegenden Anmeldung bekannte JP-OS 6550/1971 stellt sinngemäß fest, daß Verbindungen der allgemeinen Formel

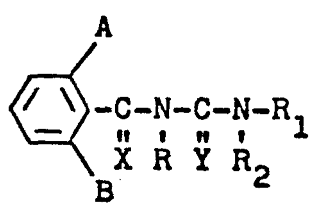

(J) ,

in der

A ein Wasserstoff-Atom, ein Halogen-Atom, ...
B ein Wasserstoff-Atom, ein Halogen-Atom, ...
X und Y ein Sauerstoff-Atom, ...
R ein Wasserstoff-Atom, ...
$R_1$ ein Wasserstoff-Atom, ... und
$R_2$ eine substituierte oder unsubstituierte Phenyl-Gruppe, ...
bezeichnen,
insektizide Aktivität besitzen.

Diese Offenlegungsschrift offenbart jedoch nicht Verbindungen der vorstehenden Formel (J), in der $R_2$ die Gruppe

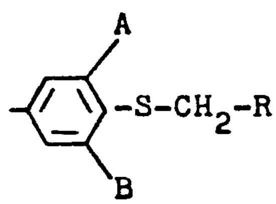

ist, wie sie in der allgemeinen Formel (I) dargestellt ist, die erstmals durch die vorliegende Erfindung speziell angegeben wird. Die vorstehende Patentschrift offenbart die folgende Verbindung:

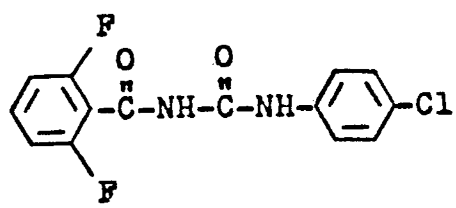

(J-1)

In Journal of Agricultural and Food Chemistry, Band 21 (3), 348 (1973), gleichfalls einer bekannten Veröffentlichung, wird über Forschungsarbeiten an 1-(2,6-Dichlorobenzoyl)-3-phenylharnstoff-Derivaten, die Synthese von nicht weniger als 157 Verbindungen dieser Reihe und die Prüfung ihrer insektiziden Aktivität berichtet. Diese Veröffentlichung offenbart jedoch ebenfalls nicht eine Verbindung, in der die N-Phenyl-Gruppe

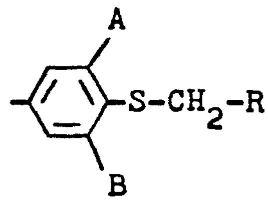

ist, wie sie in der allgemeinen Formel (I) dargestellt ist, die erstmals durch die vorliegende Erfindung speziell angegeben wird. Sie offenbart die folgende Verbindung:

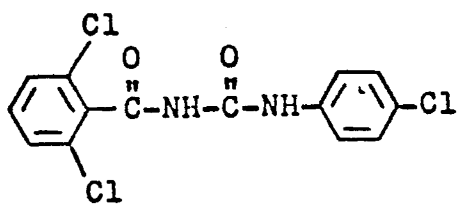

(K-1)

In Journal of Agricultural and Food Chemistry, Band 21 (6), 993 (1973) wird über eine Forschungsarbeit an 1-(2,6-disubstituiertes Benzoyl)-3-phenylharnstoff, insbesondere über den Effekt der Acyl-Gruppe auf dessen insektizide Aktivität, berichtet. Diese Veröffentlichung offenbart nicht eine Verbindung, in der die N-Phenyl-Gruppe

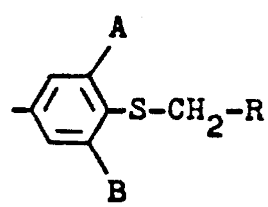

ist, wie sie in der allgemeinen Formel (I) dargestellt ist, die erstmals durch die vorliegende Erfindung speziell angegeben wird. Sie offenbart die folgende Verbindung:

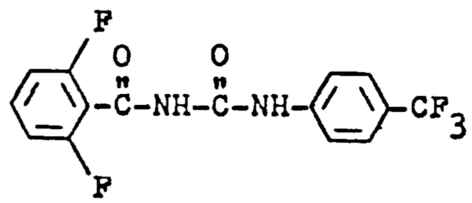 (L-1)

Die JP-OS 89646/1977, gleichfalls eine bekannte Veröffentlichung, stellt sinngemäß fest, daß N-Phenyl-N'-benzoyl-harnstoffe der allgemeinen Formel

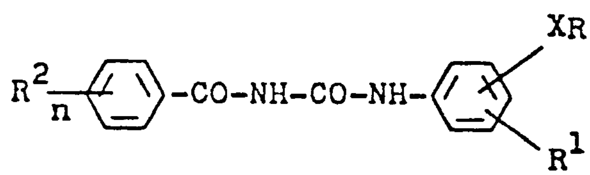 (P) ,

in der

R ein Halogenoalkyl mit 1 bis 4 Kohlenstoff-Atomen,
$R^1$ ein Wasserstoff- oder Halogen-Atom,
$R^2$ ein Halogen-Atom, ...
X ein Sauerstoff- oder Schwefel-Atom und
n 0, 1 oder 2, ...
bezeichnen,
insektizide Aktivität besitzen.

Diese japanische Offenlegungsschrift offenbart jedoch nicht eine Verbindung der Formel (P), in der die N-Phenyl-Gruppe

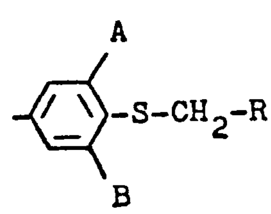

ist, wie sie in der allgemeinen Formel (I) dargestellt ist, die erstmals durch die vorliegende Erfindung speziell angegeben wird. Sie offenbart die folgende Verbindung:

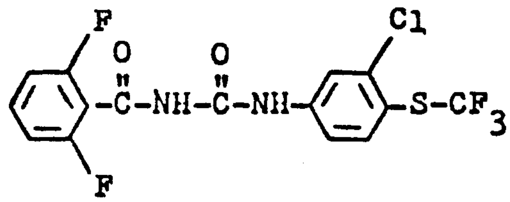

(P-1)

Die DE-OS 27 26 684 offenbart, daß Verbindungen der nachstehenden Formel

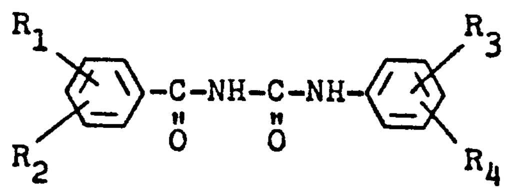

(Q)

,

in der

$R_1$ und $R_2$ Halogen,

$R_3$ ein Halogen-substituiertes $C_{1-4}$-Alkylthio, ... und

$R_4$ Wasserstoff, Halogen, ...

bezeichnen,

insektizide Aktivität besitzen.

Jedoch beschreibt diese Offenlegungsschrift nicht eine Verbindung der Formel (Q), in der $R_3$ die Gruppe $-S-CH_2-R$ ist, wie sie durch die allgemeine Formel (I) dargestellt ist, die in der vorliegenden Erfindung speziell angegeben wird. Diese DE-OS offenbart die folgende Verbindung:

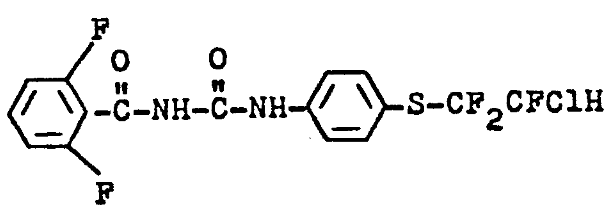

(Q-1)

Die GB-OS 2 106 499, gleichfalls eine bekannte Veröffentlichung, offenbart ein Verfahren zur Herstellung von Verbindungen der allgemeinen Formel

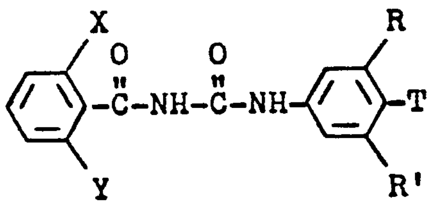

(U) ,

in der

X und Y H, F, Cl, ..,

R und R' H, Halogen, ... und

T Halogen, ... oder -ZR", worin R" Halogenoalkyl, ... und Z O oder S ist,

bezeichnen.

Jedoch offenbart diese Britische Offenlegungsschrift nicht eine Verbindung der Formel (U), in der T die Gruppe $-S-CH_2-R$ ist, wie sie durch die allgemeine Formel (I) dargestellt ist, die durch die vorliegende Erfindung speziell angegeben wird.

Gemäß der vorliegenden Erfindung wurden neue Benzoylharnstoff-Derivate der allgemeinen Formel (I) synthetisiert, die in der bisherigen Literatur nicht beschrieben sind und in denen die N-Phenyl-Gruppe

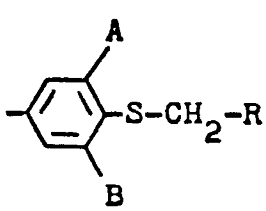

ist. Außerdem wurde gefunden, daß die Verbindungen der Formel (I) neue Benzoylharnstoff-Derivate mit sehr

guter insektizider Aktivität sind, die bei bekannten Benzoylharnstoff-Verbindungen nicht gefunden wird und von diesen nicht erwartet werden kann.

Die Verbindungen der allgemeinen Formel (I) gemäß der vorliegenden Erfindung - wie auch die bekannten Benzoylharnstoff-Verbindungen - besitzen insektizide Aktivität gegenüber Larven von Arthropoden. Gemäß der vorliegenden Erfindung wurde jedoch gefunden, daß die neuen Benzoylharnstoff-Derivate der durch die vorliegende Erfindung speziell bezeichneten allgemeinen Formel (I) eine vollständige insektizide Wirkung bei niedrigeren Dosierungen als die bekannten Benzoylharnstoff-Verbindungen aufweisen.

Es wurde gefunden, daß die Verbindungen der allgemeinen Formel (I) eine breite insektizide Wirkung gegenüber Arthropoden ausüben, insbesondere eine herausragende insektizide Wirkung gegenüber Larven von Insekten der Gattung Lepidoptera, wie im Folgenden dargelegt wird.

Das größte Kennzeichen der chemischen Struktur der Verbindungen der Formel (I) gemäß der vorliegenden Erfindung besteht darin, daß, wie in der allgemeinen Formel (I) gezeigt ist, eine fluorsubstituierte Alkyl-Gruppe mit 1 oder 2 Kohlenstoff-Atomen an die 4-Stellung der N-Phenyl-Gruppe durch die Gruppe $-S-CH_2-$ gebunden ist und ein Halogen-Atom oder eine niedere Alkyl-Gruppe in der 3-Stellung und/oder 5-Stellung der N-Phenyl-Gruppe substituiert ist(sind).

Mittels der vorliegenden Erfindung wurde erstmals diese ganz neue technische Konzeption in Verbindungen mit einer N-Benzoyl-N'-phenylharnstoff-Struktur eingeführt.

Es wurde gefunden, daß die Verbindungen mit diesem Merkmal eine sehr hohe insektizide Aktivität besitzen und rasch wirksame Effekte auslösen, die bei anderen bekannten analogen Verbindungen nicht gefunden werden.

Die Verfahren zur Gewinnung der durch die allgemeine Formel (I) bezeichneten Verbindungen gemäß der vorliegenden Erfindung läßt sich schematisch wie folgt darstellen:

Nit 172

Nit 172

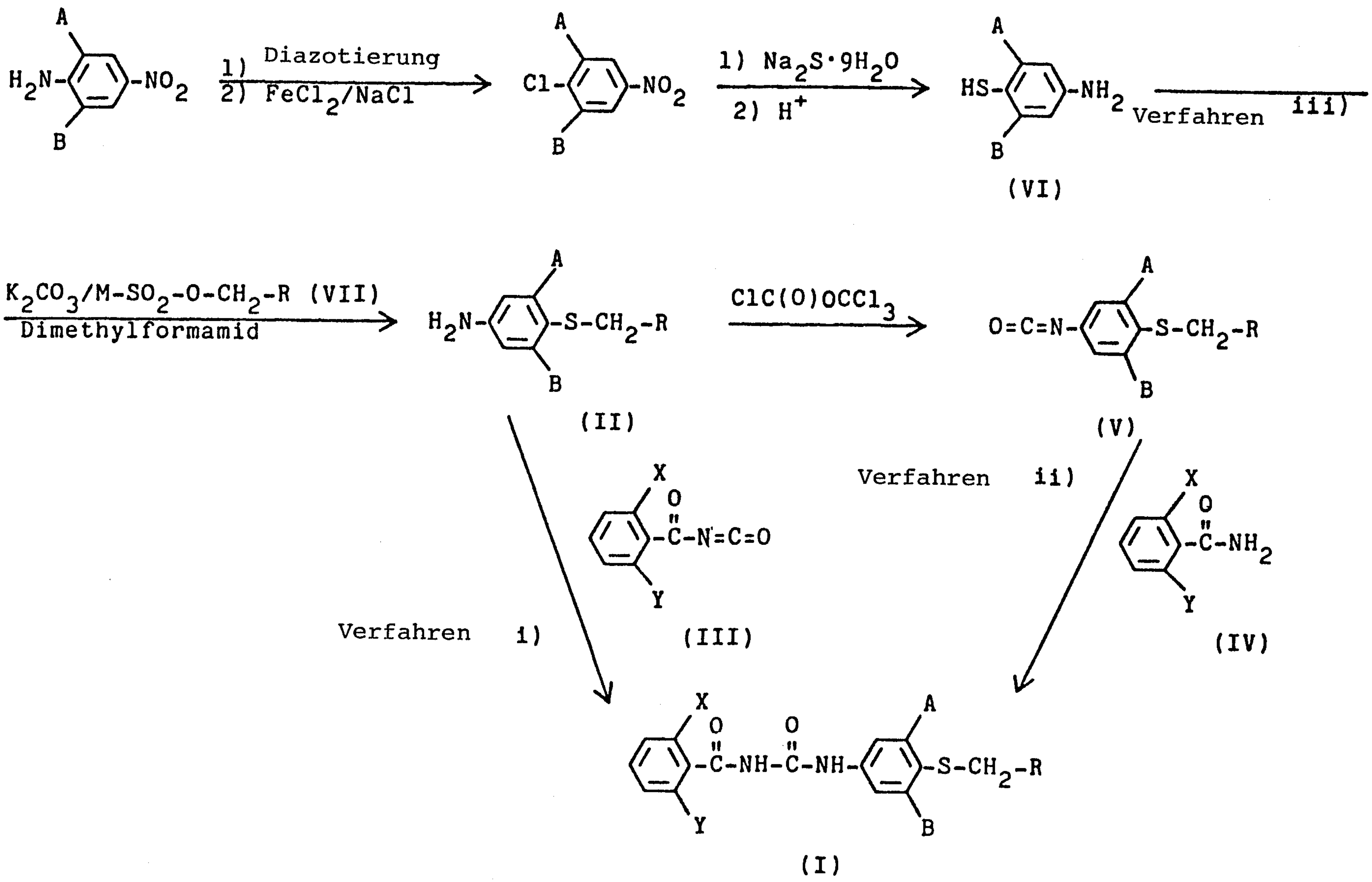
1) Diazotierung
2) FeCl$_2$/NaCl
1) Na$_2$S·9H$_2$O
2) H$^+$
(VI)
Verfahren iii)
K$_2$CO$_3$/M-SO$_2$-O-CH$_2$-R (VII)
Dimethylformamid
ClC(O)OCCl$_3$
(II)
(V)
Verfahren ii)
Verfahren i)
(III)
(IV)
(I)

In den vorstehenden Herstellungsverfahren sind die Verbindungen der allgemeinen Forml (II), ähnlich wie die Verbindungen der allgemeinen Formel (I), neue Verbindungen, die nicht in bekannten, vor dem Einreichungsdatum der vorliegenden Anmeldung erschienenen Veröffentlichungen beschrieben sind und als Zwischenprodukte von Wert sind.

Die neuen Anilin-Derivate der allgemeinen Formel (II) gemäß der vorliegenden Erfindung können mit Hilfe der oben bezeichneten Reaktionsschritte hergestellt werden. Gemäß der vorliegenden Erfindung wurde unerwartet gefunden, daß bei der Reaktion der Bildung der neuen Anilin-Derivate der allgemeinen Formel (II) aus den Mercaptoanilinen der allgemeinen Formel (VI) die Fluoroalkyl-Gruppe ($CH_2$-R) sich selektiv nicht am N-Atom sondern am S-Atom der Mercaptoaniline der allgemeinen Formel (VI) einführen läßt. Demzufolge ist es möglich, die neuen Anilin-Derivate der allgemeinen Formel (II), die gewünschten Zwischenprodukte, in hoher Ausbeute und Reinheit herzustellen.

Alternativ können die neuen Anilin-Derivate der allgemeinen Formel (II) gemäß der vorliegenden Erfindung nach dem in Bulletin of the Chemical Society of Japan, Vol. <u>50</u> (11), 3069 (1977) beschriebenen Verfahren durch Reaktion des Natriumsalzes von Nitrobenzolthiol mit Fluoroalkylmethyl-p-toluolsulfonat zur Synthese von (Fluoroalkylmethylthio)nitrobenzol und anschließende Reduktion dieser Verbindung hergestellt werden. Dieses Verfahren weist jedoch gegenüber dem erfindungsgemäßen Verfahren iii) zur Herstellung der Zwischenprodukte im Hinblick auf Ausbeute, Reaktionsschritte und die Arbeitsmethoden der Behandlung des Reaktionsproduktes

(z.B. muß in dem Alternativ-Verfahren Nitrobenzolthiol isoliert werden) Nachteile auf. Demgemäß ist das Verfahren gemäß der vorliegenden Erfindung wertvoll und technisch herausragend.

Demgemäß ist es ein Ziel der vorliegenden Erfindung, die neuen Benzoylharnstoff-Derivate der allgemeinen Formel (I), die Zwischenprodukte bei deren Herstellung der allgemeinen Formel (II), Verfahren zur Herstellung beider sowie die Verwendung der Verbindungen der allgemeinen Formel (I) als Insektizide verfügbar zu machen.

Diese und andere Ziele und Vorteile der vorliegenden Erfindung gehen aus der folgenden Beschreibung hervor.

Die aktiven Verbindungen der allgemeinen Formel (I) gemäß der vorliegenden Erfindung zeigen eine genaue Bekämpfungswirkung gegenüber schädlichen Arthropoden, ohne Phytotoxizität gegenüber Kulturpflanzen auszuüben. Die Verbindungen der vorliegenden Erfindung können zur Bekämpfung und Ausrottung eines breiten Bereichs von Schädlingen, beispielsweise gegen schädliche beißende Insekten, Pflanzenparasiten, Schädlinge auf Lagergetreide und gesundheitsgefährdende Schädlinge, eingesetzt werden.

Beispiele für die Schädlinge sind im Folgenden aufgeführt.

<u>Insekten der Ordnung Coleoptera</u>

Callosobruchus chinensis,
Sitophilus zeamais,
Tribolium castaneum,
Epilachna vigintioctomaculata,

Agriotes fuscicollis,
Anomala rufocuprea,
Leptinotarsa decemlineata,
Diabrotica spp.,
Monochamus alternatus,
Lissorhoptrus oryzophilus und
Lyctus brunneus.

Insekten der Ordnung Lepidoptera

Lymantria dispar,
Malacosoma neustria,
Pieris rapae,
Spodoptera litura,
Mamestra brassicae,
Chilo suppressalis,
Pyrausta nubilalis,
Ephestia cautella,
Adoxophyes orana,
Carpocapsa pomonella,
Agrotis fucosa,
Galleria mellonella
Plutella maculipennis und
Phyllocnistis citrella.

Insekten der Ordnung Orthoptera

Blatella germanica,
Periplaneta americana,
Gryllotalpa africana und
Locusta migratoria migratoriodes.

Insekten der Ordnung Isoptera

Deucotermes speratus und
Coptotermes formosanus.

Insekten der Ordnung Diptera

Musca domestica,
Aedes aegypti,
Hylemia platura,
Culex pipiens,
Anopheles sinensis und
Culex tritaeniorhynchus.

Auf dem Gebiet der Viehzucht sind die neuen Verbindungen gemäß der vorliegenden Erfindung wirksam gegen verschiedene schädliche und unerwünschte Insekten. Beispiele für solche Insekten sind nachstehend angegeben.

Insekten

Gastrophilus spp.,
Stomoxys spp.,
Trichodectes spp.
Rhodnius spp. und
Ctenocephalidex canis.

Die durch die allgemeine Formel (I) bezeichneten Verbindungen gemäß der vorliegenden Erfindung können mittels der folgenden Verfahren i) und ii) hergestellt werden.

20

Verfahren i)

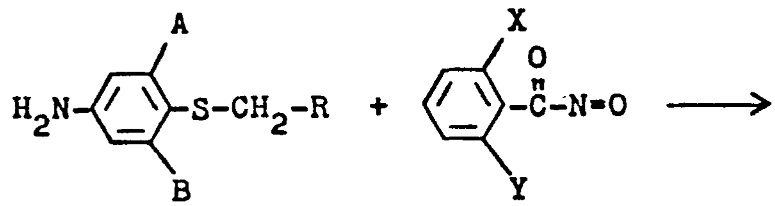

(II) (III)

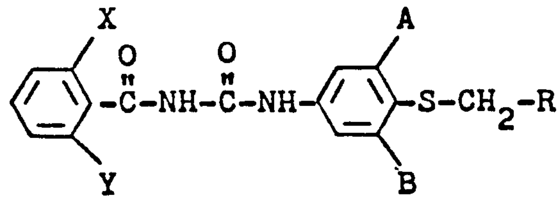

(I)

(In den Formeln haben X, Y, A, B und R die oben angegebenen Bedeutungen).

Zu speziellen Beispielen für die Verbindungen der allgemeinen Formel (II) als Ausgangsstoffe in dem Verfahren zur Herstellung der neuen Benzoylharnstoff-Derivate der allgemeinen Formel (I) gemäß der vorliegenden Erfindung, wie es im Vorstehenden schematisch dargestellt ist, zählen die nachstehenden.

3,5-Dichloro-4-(2,2,2-trifluoroethylthio)anilin,
3-Chloro-4-(2,2,2-trifluoroethylthio)anilin,
3-Chloro-4-(2,2,3,3-tetrafluoropropylthio)anilin,
3-Chloro-4-(2,2,3,3,3-pentafluoropropylthio)-anilin,
4-(2,2,2-Trifluoroethylthio)-m-toluidin,
3-Fluoro-4-(2,2,2-trifluoroethylthio)anilin,
3,5-Dichloro-4-(2,2,3,3-tetrafluoropropylthio)-anilin und
5-Chloro-4-(2,2,2-trifluoroethylthio)-m-toluidin.

Zu speziellen Beispielen für die Verbindungen der allgemeinen Formel (III), die in gleicher Weise Ausgangsstoffe sind, zählen

2-Chlorobenzoylisocyanat,
2-Fluorobenzoylisocyanat,
2,6-Dichlorobenzoylisocyanat,
2-Chloro-6-fluorobenzoylisocyanat und
2,6-Difluorobenzoylisocyanat.

Das vorstehende Verfahren wird speziell anhand des folgenden typischen Beispiels beschrieben.

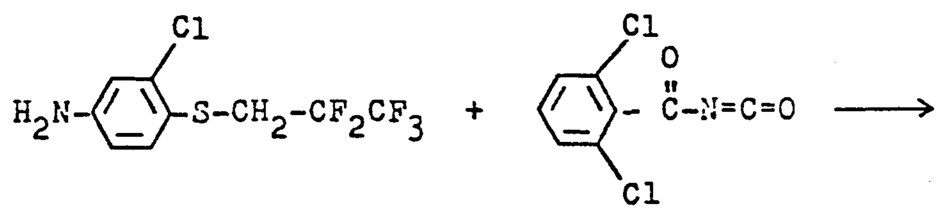

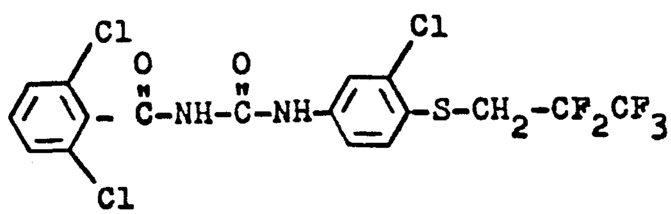

Zweckmäßigerweise wird das Verfahren zur Herstellung der vorstehenden Verbindungen gemäß der vorliegenden Erfindung unter Verwendung eines Lösungsmittels oder Verdünnungsmittels durchgeführt. Zu diesem Zweck können sämtliche inerten Lösungsmittel oder Verdünnungsmittel verwendet werden.

Beispiele für solche Lösungsmittel oder Verdünnungsmittel umfassen aliphatische, alicyclische und aromatische Kohlenwasserstoffe (die gegebenenfalls chloriert sein können), wie Hexan, Cyclohexan, Petrolether, Ligroin,

Benzol, Toluol, Xylol, Methylenchlorid, Chloroform, Kohlenstofftetrachlorid, Ethylenchlorid, Trichloroethylen und Chlorbenzol, Ether wie Diethylether, Methylethylether, Di-isopropylether, Dibutylether, Propylenoxid, Dioxan und Tetrahydrofuran, Ketone wie Aceton, Methylethylketon, Methylisopropylketon und Methylisobutylketon, Nitrile wie Acetonitril, Propionitril und Acrylnitril, Ester wie Ethylacetat und Amylacetat, Säureamide wie Dimethylformamid und Dimethylacetamid, Sulfone und Sulfoxide wie Dimethylsulfoxid und Sulfolan sowie Basen wie Pyridin.

Das obige Verfahren kann innerhalb eines breiten Temperaturbereichs durchgeführt werden. Im allgemeinen kann es bei einer Temperatur zwischen etwa -20°C und dem Siedepunkt der Mischung, zweckmäßigerweise bei einer Temperatur zwischen etwa 0°C und etwa 100°C, durchgeführt werden. Zweckmäßigerweise wird die Reaktion unter normalem Atmosphärendruck durchgeführt, jedoch ist es ebenfalls möglich, bei erhöhtem oder vermindertem Druck zu arbeiten. Vorzugsweise werden die Ausgangsstoffe in äquimolaren Mengen eingesetzt.

Verfahren ii)

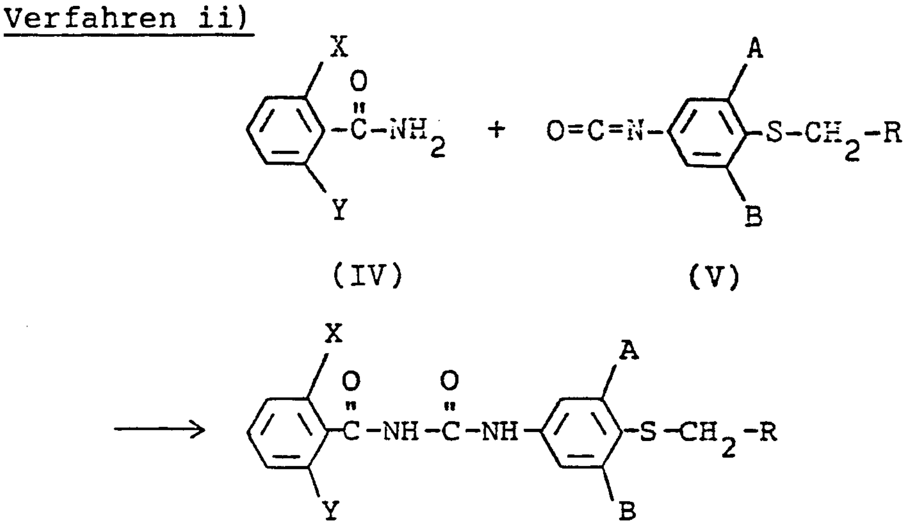

(IV) (V)

(I)

0143302

(In den Formeln haben X, Y, A, B und R die oben angegebenen Bedeutungen).

Spezielle Beispiele für die Ausgangs-Verbindung der allgemeinen Formel (IV) sind

2-Chlorobenzamid,
2-Fluorobenzamid,
2,6-Dichlorobenzamid,
2-Chloro-6-fluorobenzamid und
2,6-Difluorobenzamid.

Spezielle Beispiele für die Verbindung der allgemeinen Formel (V), die gleichfalls ein Ausgangsstoff ist, umfassen

3,5-Dichloro-4-(2,2,2-trifluoroethylthio)phenylisocyanat,
3-Chloro-4-(2,2,2-trifluoroethylthio)phenylisocyanat,
3-Chloro-4-(2,2,3,3-tetrafluoropropylthio)phenylisocyanat,
3-Chloro-4-(2,2,3,3,3-pentafluoropropylthio)-phenylisocyanat,
4-(2,2,2-Trifluoroethylthio)-m-tolylisocyanat,
3-Fluoro-4-(2,2,2-trifluoroethylthio)phenylisocyanat,
3,5-Dichloro-4-(2,2,3,3-tetrafluoropropylthio)-phenylisocyanat und
5-Chloro-4-(2,2,2-trifluoroethylthio)-m-tolylisocyanat.

Das vorstehende Verfahren wird speziell anhand des folgenden typischen Beispiels beschrieben.

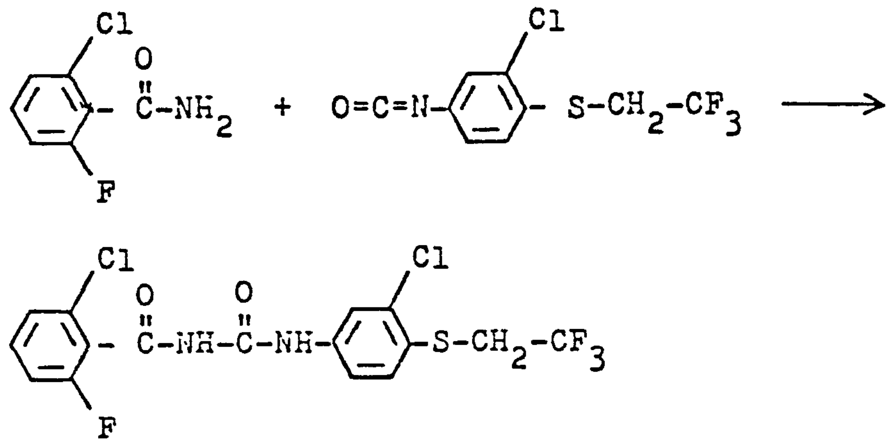

Das vorstehende Verfahren kann zweckmäßigerweise unter Verwendung der gleichen inerten Lösungsmittel oder Verdünnungsmittel durchgeführt werden, wie sie im Vorstehenden beispielhaft (Verfahren i) aufgeführt sind, wodurch das Endprodukt in hoher Reinheit und Ausbeute erhalten wird.

Zur Herstellung der aktiven Verbindungen der vorliegenden Erfindung kann die Reaktion innerhalb eines breiten Temperaturbereichs durchgeführt werden, im allgemeinen bei einer Temperatur zwischen etwa -20°C und dem Siedepunkt der Mischung, zweckmäßigerweise bei einer Temperatur zwischen etwa 0°C und etwa 100°C. Zweckmäßigerweise wird die Reaktion unter normalem Atmosphärendruck durchgeführt, jedoch ist es ebenfalls möglich, bei erhöhtem oder vermindertem Druck zu arbeiten. Vorzugsweise werden die Ausgangsstoffe in äquimolaren Mengen eingesetzt.

Die neuen, durch die allgemeine Formel (II) bezeichneten Anilin-Derivate gemäß der vorliegenden Erfindung können mittels des folgenden Verfahrens hergestellt werden.

Verfahren iii)

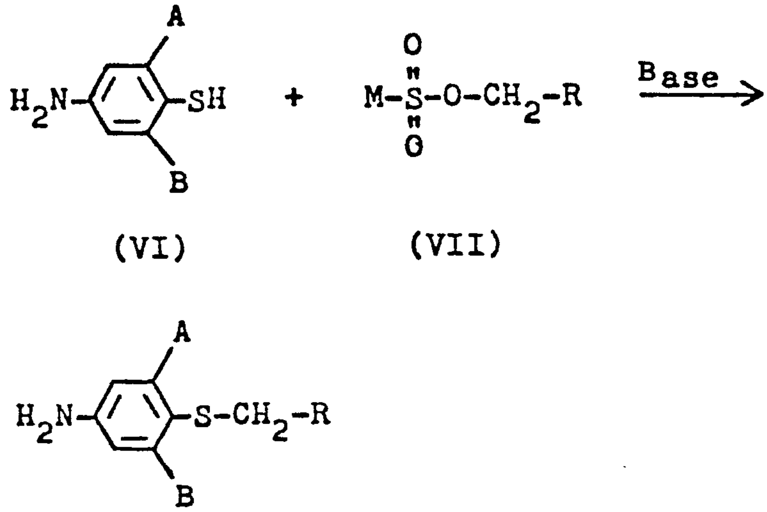


(VI) (VII)

(II)

(In den Formeln haben A, B, R und M die oben angegebenen Bedeutungen).

In dem vorstehenden Reaktionsschema können Beispiele für A, B und R die gleichen sein wie die oben angegebenen. Beispiele für M als niedere Alkyl-Gruppe sind $C_1$- bis $C_6$-, vorzugsweise $C_1$- bis $C_4$-Alkyl wie Methyl, Ethyl, n- und i-Propyl, n-, i-, sec- oder t-Butyl, vorzugsweise Methyl, oder Aryl-Gruppen, speziell eine Phenyl- oder p-Tolyl-Gruppe.

Spezielle Beispiele für die Verbindung der allgemeinen Formel (VI) als Ausgangsstoff in dem oben schematisch aufgezeigten Verfahren sind

3,5-Dichloro-4-mercaptoanilin,
3-Chloro-4-mercaptoanilin,
3-Fluoro-4-mercaptoanilin,
4-Mercapto-m-toluidin und
5-Chloro-4-mercapto-m-toluidin.

Spezielle Beispiele für die Verbindung der allgemeinen Formel (VII), die gleichfalls ein Ausgangsstoff ist, umfassen

2,2,2-Trifluoroethyl-p-toluolsulfonat,
2,2,3,3-Tetrafluoropropyl-p-toluolsulfonat und
2,2,3,3,3-Pentafluoropropyl-p-toluolsulfonat.

Die entsprechenden Methansulfonate und Benzolsulfonate können ebenfalls als Beispiele genannt werden.

Das vorstehende Verfahren wird anhand des folgenden typischen Beispiels speziell beschrieben.

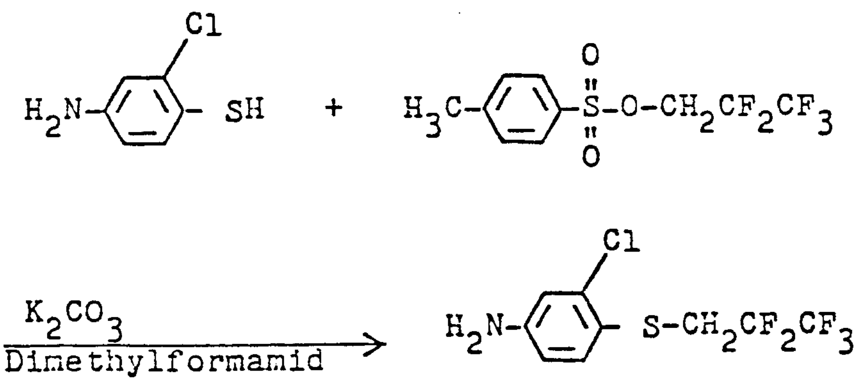


Das vorstehende Verfahren kann in Gegenwart einer Base wie Natriumcarbonat, Kaliumcarbonat, Natriumhydroxid, Kaliumhydroxid oder Natriumhydrid in einem aprotischen, polaren Lösungsmittel durchgeführt werden, wodurch das gewünschte Endprodukt in hoher Reinheit und Ausbeute erhalten wird. Beispiele für solche Lösungsmittel sind Dimethylformamid, Dimethylacetamind, Dimethylsulfoxid, Sulfolan, N-Methyl-2-pyrrolidon und Hexamethylphosphoramid. Tertiäre Alkohole wie tert-Butanol, die Protonenlösungsmittel sind, können ebenfalls verwendet werden.

Nit 172

Das obige Verfahren kann innerhalb eines breiten Temperaturbereichs, beispielsweise bei einer Temperatur zwischen etwa 0°C und etwa 120°C, zweckmäßigerweise bei einer Temperatur zwischen etwa 20°C und etwa 80°C, durchgeführt werden. Zweckmäßigerweise wird die Reaktion unter normalem Atmosphärendruck durchgeführt, jedoch ist es ebenfalls möglich, bei erhöhtem oder vermindertem Druck zu arbeiten. Vorzugsweise werden die Ausgangsstoffe in äquimolaren Mengen eingesetzt.

Die Ausgangs-Verbindung der allgemeinen Formel (VI) in dem obigen Verfahren iii) kann aus einem Halogenonitrobenzol wie 4-Chloronitrobenzol

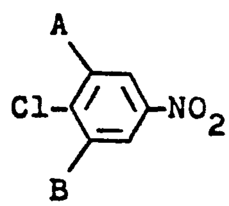

nach einer bekannten Arbeitsweise /‾Journal of the American Chemical Society, Vol. 71, 1747 (1949)_7 gewonnen werden (vgl. auch das Reaktionsschema im Vorstehenden).

Einige der in dem Verfahren iii) eingesetzten Ausgangs-Verbindungen der allgemeinen Formel (VI) sind gegenüber einer Oxidationsreaktion empfindlich. Beispielsweise ist 3,5-Dichloro-4-mercaptoanilin empfindlich gegen eine Oxidationsreaktion. In einem solchen Falle wird das Verfahren iii) vorzugsweise unter einem inerten Gas wie Stickstoff-Gas durch Einwirkung von Kaliumcarbonat oder Kalium-tert-butoxid auf eine Lösung der Verbindung in dem oben genannten Reaktionslösungsmittel oder durch Einwirkung von Natriumhydrid auf das Mercaptoanilin durchgeführt, aus dem das Produkt während seiner Synthese durch Oxidation gebildet wird (ein dem Mercaptoanilin entsprechendes Disulfid, d.h.

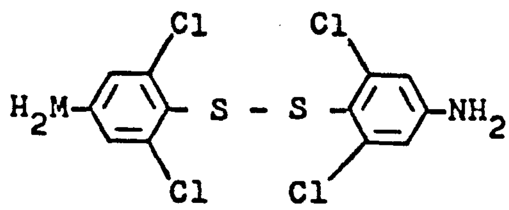

wurde nicht isoliert).

Die Verbindung der allgemeinen Formel (VII), die in gleicher Weise ein Ausgangsstoff in dem Verfahren iii) ist, kann aus einem Sulfonylchlorid und einem Fluoroalkanol mit Hilfe bekannter Arbeitsweisen /¯Journal of the American Chemical Society, Vol. 86, 4645 (1964); ibid. Vol. 75, 5978 (1953); und The Journal of Organic Chemistry Vol. 35, 3195 (1970)_7 gewonnen werden. Diese Verfahrensweisen sind im Hinblick auf die erhältliche Menge, die Komplexität der Isolierung der Endprodukt-Verbindung und die Kosten nicht zufriedenstellend. Zur Lösung dieser Probleme macht es die vorliegende Erfindung möglich, die oben genannte Verbindung leicht in hoher Reinheit und Ausbeute aus Sulfonylchlorid, einem Fluoroalkanol und Natriumhydroxid unter Verwendung eines Phasentransfer-Katalysators wie Triethylbenzylammoniumchlorid oder Tetramethylammoniumbromid zu gewinnen.

In den vorstehenden Formeln (I) bis (VII) haben die Reste X, Y, A, B und R die folgenden bevorzugten Bedeutungen:

Die Halogene X und Y können gleich oder verschieden und Fluor, Chlor, Brom oder Iod sein, vorzugsweise Fluor, Chlor und Brom und besonders bevorzugt Fluor oder Chlor.

Die Halogene A und B können gleich oder verschieden und Fluor, Chlor, Brom oder Iod sein, vorzugsweise Fluor und Chlor und besonders bevorzugt Chlor.

Die niederen Alkyl-Gruppen A und B können gleich oder verschieden und geradkettige oder verzweigte Alkyl-Gruppen mit 1 bis 8, vorzugsweise mit 1 bis 6 und besonders bevorzugt mit 1 bis 4 Kohlenstoff-Atomen sein, z.B. Methyl, Ethyl, n- und i-Propyl, n-, i-, sec- und t-Butyl. Vor allen anderen bevorzugt ist die Methyl-Gruppe.

Die fluorosubstituierte Alkyl-Gruppe R hat 1 oder 2 Kohlenstoff-Atome und 1 bis 5 Fluor-Atome, z.B. Fluoromethyl, Difluoromethyl, 1,2-Difluoroethyl und vorzugsweise Trifluoromethyl, Pentafluoroethyl und 1,1,2,2-Tetrafluoroethyl.

Als insektizides Mittel kann die durch die allgemeine Formel (I) bezeichnete Verbindung gemäß der vorliegenden Erfindung unmittelbar nach dem Verdünnen mit Wasser oder aber in Form verschiedenartiger Formulierungen zur Anwendung gebracht werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden diese verschiedenen Formulierungen entweder unmittelbar oder nach dem Verdünnen mit Wasser auf die gewünschte Konzentration eingesetzt.

Beispiele für die hierin erwähnten landwirtschaftlich unbedenklichen Hilfsstoffe sind Verdünnungsmittel (Lösungsmittel, Streckmittel, Träger), oberflächenaktive

Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe /‾z.B. n-Hexan, Petrolether, Erdöl-Fraktionen (z.B. Paraffinwachse, Kerosin, Leichtöle, Mittelöle und Schweröle), Benzol, Toluol und Xylol_7, halogenierte Kohlenwasserstoffe (z.B. Methylenchlorid, Kohlenstofftetrachlorid, Ethylenchlorid, Ethylendibromid, Chlorbenzol und Chloroform), Alkohole (z.B. Methanol, Ethanol, Propanol und Ethylenglycol), Ether (z.B. Diethylether, Ethylenoxid und Dioxan), Alkohol-ether (z.B. Ethylenglycol-monomethylether), Ketone (z.B. Aceton und Isophoron), Ester (z.B. Ethylacetat und Amylacetat), Amide (z.B. Dimethylformamid und Dimethylacetamid) und Sulfoxide (z.B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z.B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylschwefelsäureester (z.B. Natriumlaurylsulfat), Arylsulfonsäuren

(z.B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z.B. Laurylamin, Stearyltrimethylammoniumchlorid und Alkyldimethylbenzylammoniumchloride) und Polyoxyethylenalkylamine, nicht-ionische oberflächenaktive Mittel wie Polyoxyethylenglycolether (z.B. Polyoxyethylenalkylarylether und deren Kondensationsprodukte), Polyoxyethylenglycolester (z.B. Polyoxyethylenfettsäureester) und Ester mehrwertiger Alkohole (z.B. Polyoxyethylensorbitan-monolaurat) sowie amphotere oberflächenaktive Mittel.

Beispiele für andere Hilfsstoffe umfassen Stabilisatoren, Haftmittel (z.B. landwirtschaftliche Seifen, Casein-Kalk, Natriumalginat, Polyvinylalkohol, Haftmittel vom Vinylacetat-Typ und Acryl-Haftmittel), Aerosol-Treibmittel (z.B. Trichlorofluoromethan, Dichlorofluoromethan, 1,2,2-Trichloro-1,1,2-trifluoroethan, Chlorbenzol, verflüssigtes Erdgas (LNG) und niedere Ether), die Verbrennung steuernde Mittel für Räuchermittel (z.B. Nitrite, Zink-Pulver und Dicyandiamid), sauerstoff-abgebende Mittel (z.B. Chlorate), wirkungsverlängernde Mittel, Dispersions-Stabilisatoren (z.B. Casein, Tragant, Carboxymethylcellulose (CMC) und Polyvinylalkohol (PVA)) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öl-Präparate,

benetzbare Pulver, lösliche Pulver, Suspensionen, Stäubemittel, Granulate, pulvrige Präparate, Räuchermittel, Tabletten, Aerosole, Pasten und Kapseln.

Die insektiziden Mittel gemäß der vorliegenden Erfindung können etwa 0,1 bis etwa 95 Gew.-%, vorzugsweise von etwa 0,5 bis etwa 90 Gew.-%, des vorerwähnten Wirkstoffes enthalten.

Für den praktischen Gebrauch beträgt die geeignete Menge der aktiven Verbindung in den vorgenannten verschiedenartigen Formulierungen und gebrauchsfertigen Präparaten im allgemeinen etwa 0,0001 bis etwa 20 Gew.-%, vorzugsweise etwa 0,005 bis etwa 10 Gew.-%.

Der Gehalt des Wirkstoffs kann in geeigneter Weise je nach der Art der Formulierung, dem Verfahren, Zweck, der Zeit und dem Ort seiner Anwendung sowie dem Zustand des Auftretens der zu bekämpfenden Schädlinge variiert werden.

Erforderlichenfalls können die Verbindungen gemäß der vorliegenden Erfindung weiterhin auch in Kombination mit anderen Agrochemikalien verwendet werden, beispielsweise mit anderen Insektiziden, Fungiziden, Mitiziden, Nematiziden, Anti-Virus-Mitteln, Herbiziden, Pflanzen-Wachstumsregulatoren und Lockstoffen /¯z.B. Organophosphat-Verbindungen, Carbamat-Verbindungen, Dithio(oder thiol)carbamat-Verbindungen, Organochlor-Verbindungen, Dinitro-Verbindungen, organischen Schwefel- oder metallorganischen Verbindungen, Antibiotika, substituierten Diphenylether-Verbindungen, Harnstoff-Verbindungen und Triazin-Verbindungen_7 und/oder Düngemitteln.

Den im Vorstehenden bezeichneten Wirkstoff enthaltende verschiedenartige Mittel und gebrauchsfertige Präparate können mittels verschiedener Verfahren zur Anwendung gebracht werden, wie sie allgemein für das Aufbringen von Agrochemikalien gebräuchlich sind, beispielsweise durch Sprühen (Versprühen von Flüssigkeiten, Vernebeln, Zerstäuben, Stäuben, Streuen von Granulat, Wasser-Oberflächenbehandlung, Gießen etc.), Räuchern, Bodenbehandlung (Vermischen mit dem Boden, Spritzen, Bedampfen, Gießen etc.), Oberflächen-Anwendung (z.B. Beschichten, Aufbringen in Form von Bändern, Pulverbeschichten, Bedecken etc.), Eintauchen und Ködern. Sie können auch mittels des sogenannten Ultra-Low-Volume-Sprühverfahrens aufgebracht werden. Nach diesem Verfahren kann der Wirkstoff sogar in einer Konzentration von 100 % eingearbeitet sein.

Die Aufwandmenge pro Flächeneinheit beträgt beispielsweise etwa 0,03 bis etwa 10 kg/ha, vorzugsweise etwa 0,3 bis etwa 6 kg/ha. In besonders gelagerten Fällen können oder sollten sogar die Aufwandmengen außerhalb des angegebenen Bereichs liegen.

Gemäß der vorliegenden Erfindung kann ein insektizides, Mittel zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung macht weiterhin ein Verfahren zur Bekämpfung von Schädlingen verfügbar, das darin

besteht, daß eine Verbindung der allgemeinen Formel (I) allein oder in Form einer Mischung mit einem Verdünnungsmittel (einem Lösungsmittel und/ oder einem Streckmittel und/oder einem Träger) und/oder einem oberflächenaktiven Mittel und, falls weiterhin erforderlich, einem Stabilisator, einem Haftmittel, einem synergistischen Mittel etc. auf Schädlinge und/oder deren Lebensraum oder den Ort ihres Auftretens aufgebracht wird.

Die vorliegende Erfindung wird durch die folgenden Beispiele im einzelnen erläutert, ist jedoch nicht auf diese speziellen Beispiele allein beschränkt.

Beispiel 1 (Synthesebeispiel)

Oxalylchlorid (4 g) wurde tropfenweise zu einer Mischung von 2-Chloro-6-fluorobenzamid (3,47 g) und Methylenchlorid (40 ml) hinzugefügt. Nach Beendigung der heftigen Reaktion wurde die Reaktionsmischung 5 h unter Rückfluß erhitzt. Die niedrigsiedenden Stoffe wurden unter vermindertem Druck abgedampft, und dem Rückstand wurde Toluol (40 ml) zugesetzt. Wiederum wurden die niedrigsiedenden Stoffe unter vermindertem Druck abgedampft. Der Rückstand (2-Chloro-6-fluorobenzoylisocyanat) wurde in Toluol (40 ml) gelöst, und die Lösung wurde tropfenweise bei 20°C bis 25°C zu einer Lösung aus 3,5-Dichloro-4-(2,2,2-trifluoroethylthio)-anilin (6 g) und Toluol (30 ml) hinzugegeben. Nach der Zugabe wurde die Mischung 6 h bei 25°C bis 35°C gerührt. Das Toluol wurde unter vermindertem Druck abgedampft. Der Rückstand wurde aus Ethanol umkristallisiert, wonach der gewünschte 1-(2-Chloro-6-fluorobenzoyl)-3-/¯3,5-dichloro-4-(2,2,2-trifluoroethylthio)phenyl_7harnstoff (7,3 g) der nachstehenden Formel erhalten wurde. Schmp. 195-196°C.

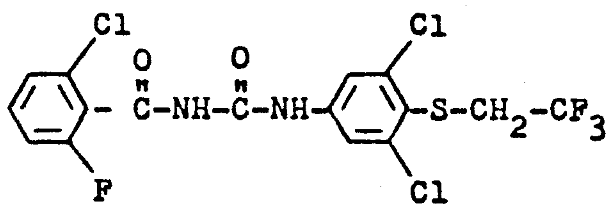

(Verbindung Nr. 1)

Beispiel 2 (Synthesebeispiel)

Eine Mischung aus 2-Chloro-6-fluorobenzamid (3,47 g), 3-Chloro-4-(2,2,2-trifluoroethylthio)phenylisocyanat (5,4 g) und Xylol (80 ml) wurde 24 h unter Rückfluß erhitzt. Die Reaktionsmischung wurde gekühlt. Die erhaltenen Kristalle wurden durch Filtration gesammelt und aus Ethanol umkristallisiert, wonach 6,4 g 1-(2-Chloro-6-fluorobenzoyl)-3-/‾3-Chloro-4-(2,2,2-trifluoroethylthio)phenyl_7harnstoff erhalten wurde. Schmp. 183,5-184,5°C.

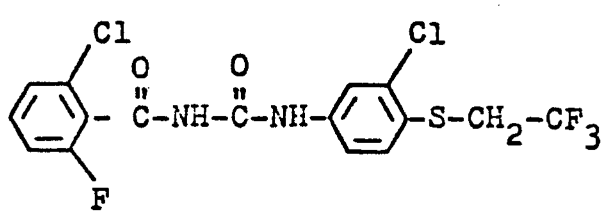

(Verbindung Nr. 2)

Nach im wesentlichen der gleichen Verfahrensweise wie in den Beispielen 1 und 2 wurden die in Tabelle 1 aufgeführten Verbindungen synthetisiert.

Tabelle 1

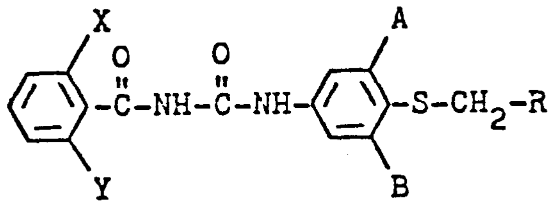

| Verbindung | X | Y | A | B | R | Physikal. Konstante: Schmp. (°C) |
|---|---|---|---|---|---|---|
| 3 | Cl | Cl | Cl | H | $-CF_2CHF_2$ | 179 - 180 |
| 4 | Cl | Cl | Cl | H | $-CF_2CF_3$ | 232 - 240 |
| 5 | Cl | F | $-CH_3$ | H | $-CF_3$ | 159 - 160 |
| 6 | F | F | Cl | Cl | $-CF_3$ | 190 - 191 |
| 7 | Cl | H | Cl | Cl | $-CF_3$ | 187 - 190 |
| 8 | F | H | Cl | Cl | $-CF_3$ | 152 - 158 |
| 9 | Cl | Cl | Cl | Cl | $-CF_3$ | 202 - 207 |
| 10 | F | F | Cl | Cl | $-CF_2CHF_2$ | 187 - 188 |
| 11 | Cl | F | Cl | Cl | $-CF_2CHF_2$ | 191 - 192 |
| 12 | Cl | F | Cl | $-CH_3$ | $-CF_3$ | 209 - 210 |
| 13 | F | F | Cl | $-CH_3$ | $-CF_3$ | 184 - 186 |

Beispiel 3 (Synthesebeispiel für die Zwischenprodukte)

3-Chloro-4-mercaptoanilin (71 g) wurde in N,N-Dimethylformamid (250 ml) gelöst, und im Stickstoff-Strom wurde Kalium-tert-butoxid (49,4 g) zugegeben. Die Mischung wurde 10 min gerührt. Während 2,2,2-Trifluoroethyl-p-toluolsulfonat nach und nach in kleinen Anteilen bei

20°C bis 25°C zu der entstandenen Lösung hinzugefügt wurde, wurde die Mischung 1 h bei 25°C bis 35°C und weiter 6 h bei 75°C bis 85°C gerührt. N,N-Dimethylformamid wurde abgedampft, und Toluol (300 ml) und eine 5-proz. wäßrige Kaliumhydroxid-Lösung (150 ml) wurden dem Rückstand zugesetzt. Die Mischung wurde gerührt und dann stehen gelassen. Die organische Schicht wurde abgetrennt, mit Wasser gewaschen und über wasserfreiem Natriumsulfat getrocknet. Toluol wurde abgedampft, und der Rückstand wurde unter vermindertem Druck destilliert, wodurch das gewünschte 3-Chloro-4-(2,2,2-trifluoroethylthio)anilin (82 g) der nachstehenden Formel erhalten wurde. Sdp. 130-131°C/1,07 mbar(0,8 mmHg) erhalten wurde.

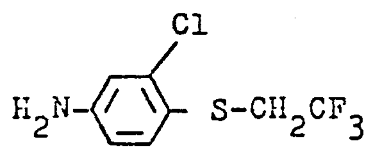

(Verbindung Nr. II-1)

Nach im wesentlichen der gleichen Arbeitsweise wie in Beispiel 3 wurden die in Tabelle 2 aufgeführten Verbindungen synthetisiert.

0143302



Tabelle 2

| Verbindung Nr. | Verbindung |
| --- | --- |
| II-2 | 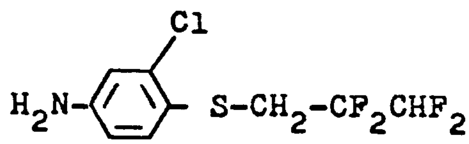 Sdp. 136 - 138°C/ 1,07 mbar (0,8 mmHg) |
| II-3 | 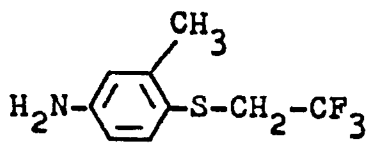 Sdp. 106 - 108°C/1,07 mbar (0,8 mmHg) |
| II-4 | 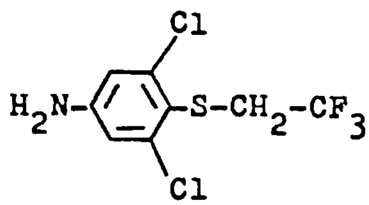 Schmp. 103 - 104°C |
| II-5 | 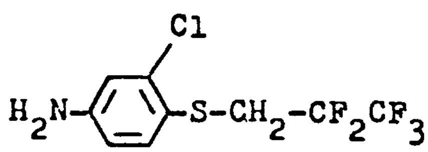 |
| II-6 | 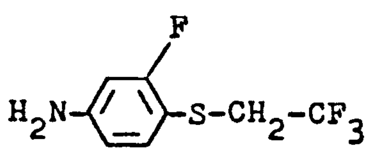 |

Fortsetzung Tabelle 2

| Verbindung Nr. | Verbindung |
|---|---|
| II-7 | 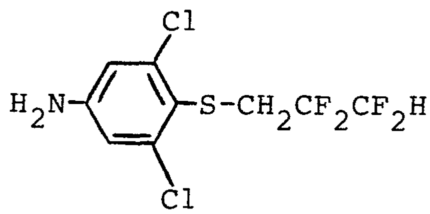 Sdp. 120 - 122°C/0,5 Torr |
| II-8 | 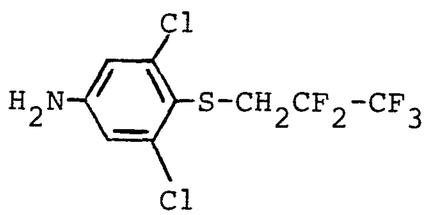 (Öl) |
| II-9 | 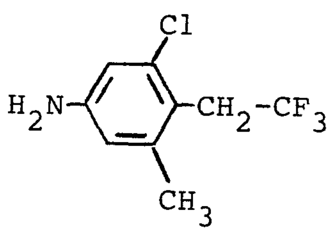 Schmp. 99 - 101°C |

3-Chloro-4-mercaptoanilin und 2,2,2-Trifluoroethyl-p-toluolsulfonat, als Rohstoffe in Beispiel 3 eingesetzt, können wie in den folgenden Bezugsbeispielen synthetisiert werden.

Nit 172

Bezugsbeispiel 1

Eine Mischung aus 3,4-Dichloronitrobenzol (156 g), Natriumsulfid-nonahydrat (480 g) und 2 l Wasser wurde 8 h unter Rückfluß erhitzt. Die Reaktionsmischung wurde abgekühlt und mit Ether (400 ml) gerührt. Die wäßrige Schicht wurde abgetrennt und mit Natriumchlorid gesättigt. Unter Rühren wurden 240 g Essigsäure tropfenweise hinzugefügt. Die Mischung wurde zur Gewinnung des gewünschten 3-Chloro-4-mercaptoanilins mit drei 400 ml-Portionen Ether extrahiert. Die Extrakte wurden über wasserfreiem Natriumsulfat getrocknet. Der Ether wurde abgedampft, und der Rückstand wurde unter vermindertem Druck destilliert, wonach 100 g des gewünschten 3-Chloro-4-mercaptoanilins erhalten wurden. Sdp. 173-174°C/ 26,7 mbar (20 mmHg).

Bezugsbeispiel 2

Eine wäßrige Lösung aus 42 g Natriumhydroxid und 100 g Wasser wurde tropfenweise bei 20°C bis 35°C zu einer Lösung aus 2,2,2-Trifluoroethanol (105 g), Toluol (2000 ml), p-Toluolsulfonylchlorid (190,5 g) und Triethylbenzylammoniumchlorid (2,5 g) gegeben. Die Mischung wurde 4 h bei 50°C bis 60°C gerührt, und Toluol (800 ml) wurde zugesetzt. Die Mischung wurde stehen gelassen. Die organische Schicht wurde abgetrennt, mit Wasser gewaschen und über wasserfreiem Natriumsulfat getrocknet. Die niedrigsiedenden Stoffe wurden unter vermindertem Druck abgedampft. Zu dem Rückstand wurden 600 ml n-Hexan hinzugefügt, und unter heftigem Rühren wurde die Mischung mit Eis gekühlt. Das gewünschte 2,2,2-Trifluoroethyl-p-toluolsulfonat (250 g) wurde in Form von Kristallen erhalten. Schmp. 40-41°C.

Beispiel 4 (Benetzbares Pulver)

15 Teile der Verbindung Nr. 1 der Erfindung, 80 Teile eines Gemisches (1:5) aus pulvriger Diatomeenerde und Tonpulver, 2 Teile Natriumalkylbenzolsulfonat und 5 Teile Natriumalkylnaphthalinsulfonat/Formaldehyd-Kondensat werden pulverisiert und zu einem benetzbaren Pulver vermischt. Dieses wird mit Wasser verdünnt und auf Schädlinge und/oder ihren Lebensraum aufgesprüht.

Beispiel 5 (Emulgierbares Konzentrat)

30 Teile der Verbindung Nr. 2 der Erfindung, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Schädlinge und/oder ihren Lebensraum aufgesprüht.

Beispiel 6 (Stäubemittel)

2 Teile der Verbindung Nr. 3 der Erfindung und 98 Teile Tonpulver werden pulverisiert und gemischt, wodurch ein Stäubemittel hergestellt wird. Dieses wird über Schädlingen und/oder ihrem Lebensraum ausgestreut.

Beispiel 7 (Stäubemittel)

Die Verbindung Nr. 4 der Erfindung (1,5 Teile), 0,5 Teile Isopropylhydrogenphosphat (PAP) und 98 Teile Tonpulver werden pulverisiert und gemischt, wodurch ein Stäubemittel hergestellt wird. Dieses wird über Schädlingen und/oder ihrem Lebensraum ausgestreut.

Beispiel 8 (Granulat)

Wasser (25 Teile) wird zu einer Mischung aus 10 Teilen der Verbindung Nr. 5 der Erfindung, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet, das dann bei 40°C bis 50°C getrocknet wird. Das erhaltene Granulat wird über Schädlingen und/oder ihrem Lebensraum ausgestreut.

Beispiel 9 (Biologischer Test)

Test mit Larven von Spodoptera litura:

Herstellung der Test-Chemikalie:

| | | |
|---|---|---|
| Lösungsmittel: | Xylol | 3 Gew.-Teile |
| Emulgator: | Polyoxyethylen-alkylphenylether | 1 Gew.-Teil |

Zur Herstellung von geeigneten Wirkstoff-Präparaten wurde 1 Gew.-Teil der betreffenden aktiven Verbindungen mit der angegebenen Menge Lösungsmittel, die die angegebene Menge Emulgator enthielt vermischt. Die Mischung wurde mit Wasser auf eine vorher festgesetzte Konzentration verdünnt.

Test-Verfahren:

Blätter süßer Kartoffeln wurden in die wäßrige Verdünnung jedes der Wirkstoffe getaucht. Nach dem Trocknen an der Luft wurden die Blätter in Petrischalen von 9 cm Durchmesser gelegt. 10 Larven im 3. Stadium von Spodoptera litura wurden in die Petrischalen eingesetzt, und die Petrischalen wurden in einen Raum mit konstant

gehaltener Temperatur von 28°C gestellt. 7 Tage später wurde der Wert der letalen Konzentration ($LC_{100}$-Wert) (ppm) berechnet.
Die Ergebnisse sind in Tabelle 3 aufgeführt.

Tabelle 3

| Verbindung Nr. | $LC_{100}$ (ppm) |
|---|---|
| 1 | 1 |
| 2 | 10 |
| 5 | 10 |
| 6 | 1 |
| 8 | 1 |
| 9 | 1 |
| 10 | 1 |
| 11 | 1 |
| 12 | 1 |
| 13 | 1 |
| Vergleichs-Verbindung | |
| J-1 | > 20 |
| K-1 | > 20 |
| L-1 | > 20 |
| P-1 | > 20 |
| Q-1 | > 20 |
| W-1 | > 20 |

Anmerkungen:

1. Die Verbindungs-Nummern sind die gleichen wie oben erwähnt.
2. Die Vergleichs-Verbindungen J-1, K-1, L-1, P-1, Q-1 und W-1 stellen die folgenden Verbindungen dar:

J-1: 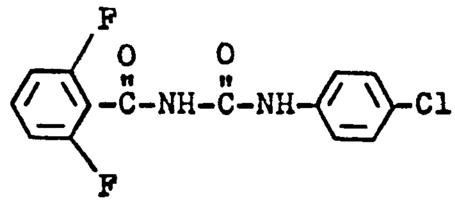

K-1: 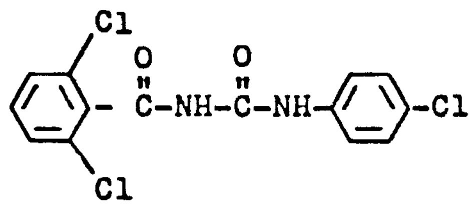

L-1: 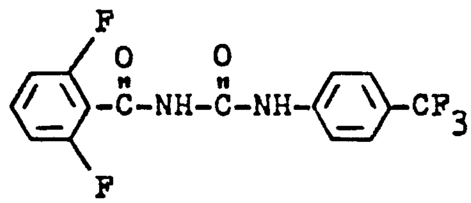

P-1: 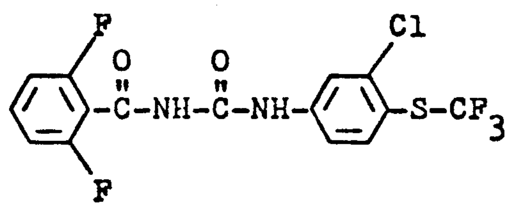

Q-1: 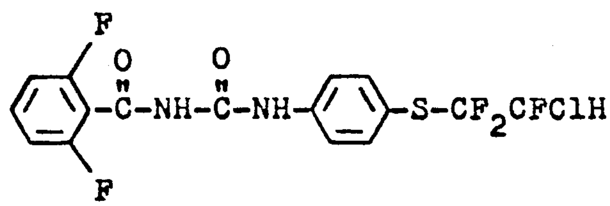

W-1: 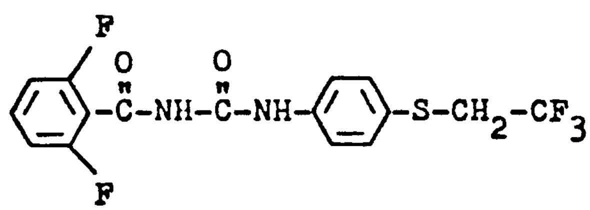

Beispiel 10 (Biologischer Test)
Test mit Larven von Plutella maculipennis:

Test-Verfahren:

Kohl-Blätter wurden in die wäßrige Verdünnung mit vorher festgelegter Konzentration jedes der Wirkstoffe getaucht, die wie in Beispiel 9 hergestellt worden waren. Nach dem Eintrocknen der Chemikalie an der Luft wurden die Blätter in Petrischalen von 9 cm Durchmesser gelegt, und 10 Larven im 2. Stadium von Plutella maculipennis wurden in die Petrischalen eingesetzt. Die Petrischalen wurden dann in einem Raum mit konstanter Temperatur von 23°C belassen, und 7 Tage später wurde der $LC_{100}$-Wert (ppm) berechnet.
Die Ergebnisse sind in Tabelle 4 aufgeführt.

Tabelle 4

| Verbindung Nr. | $LC_{100}$ (ppm) |
|---|---|
| 1 | 1 |
| 2 | 5 |
| 5 | 2 |
| 6 | 0,5 |
| 9 | 2 |
| Vergleichs-Verbindung | |
| J-1 | > 20 |
| K-1 | > 20 |
| L-1 | > 20 |
| Q-1 | > 20 |
| W-1 | > 20 |

## Patentansprüche

1. Neue Benzoylharnstoff-Derivate der allgemeinen Formel (I)

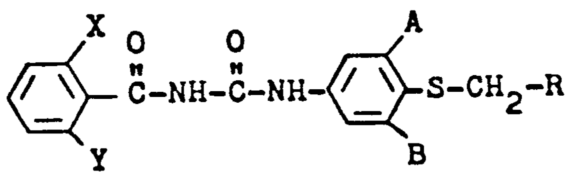

(I)

,

in der

X und Y jeweils ein Wasserstoff- oder Halogen-Atom bezeichnen, mit der Maßgabe, daß X und Y nicht gleichzeitig ein Wasserstoff-Atom bezeichnen,

A und B jeweils ein Wasserstoff-Atom, eine niedere Alkyl-Gruppe oder ein Halogen-Atom bezeichnen, mit der Maßgabe, daß A und B nicht gleichzeitig ein Wasserstoff-Atom bezeichnen, und

R eine fluoro-substituierte Alkyl-Gruppe mit 1 bis 2 Kohlenstoff-Atomen bezeichnet.

2. Verbindung nach Anspruch 1, dadurch gekennzeichnet, daß X ein Halogen-Atom und Y ein Wasserstoff-Atom sind oder X und Y identische oder verschiedene Halogen-Atome sind.

3. Verbindung nach Anspruch 1 und 2, dadurch gekennzeichnet, daß A ein Halogen-Atom und B ein Wasserstoff-Atom sind oder A und B identische oder verschiedene Halogen-Atome sind.

4. Benzoylharnstoff-Derivate der Formeln

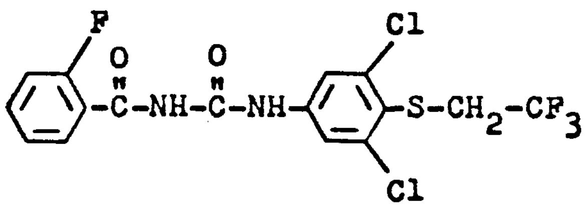

,

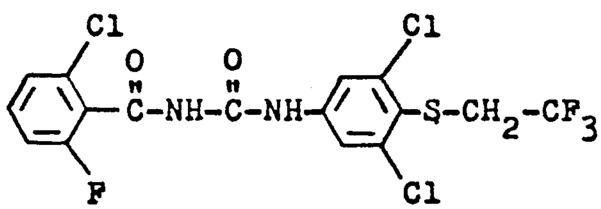

,

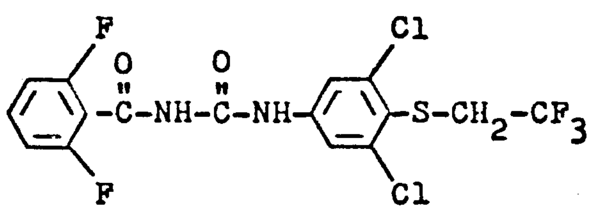

,

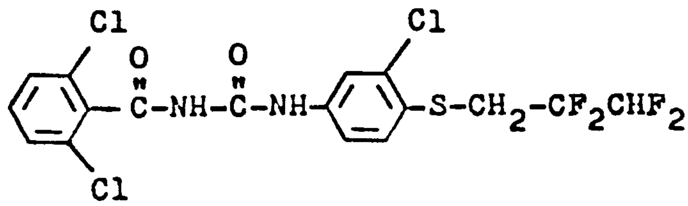

und

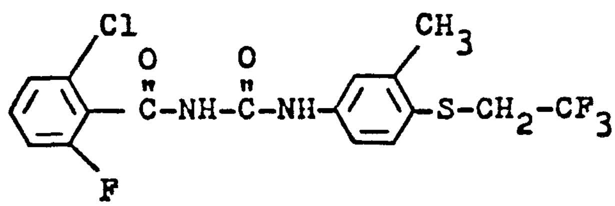

.

5. Verfahren zur Herstellung neuer Benzoylharnstoff-Derivate der allgemeinen Formel (I)

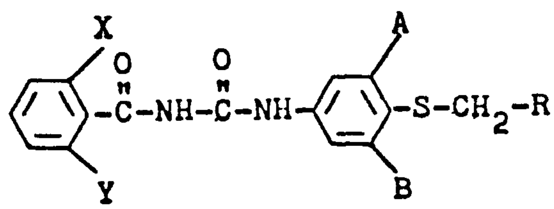

(I)

,

in der

X und Y jeweils ein Wasserstoff- oder Halogen-Atom bezeichnen, mit der Maßgabe, daß X und Y nicht gleichzeitig ein Wasserstoff-Atom bezeichnen,

A und B jeweils ein Wasserstoff-Atom, eine niedere Alkyl-Gruppe oder ein Halogen-Atom bezeichnen, mit der Maßgabe, daß A und B nicht gleichzeitig ein Wasserstoff-Atom bezeichnen, und

R eine fluoro-substituierte Alkyl-Gruppe mit 1 bis 2 Kohlenstoff-Atomen bezeichnet,

dadurch gekennzeichnet, daß

i) eine Verbindung der allgemeinen Formel (II)

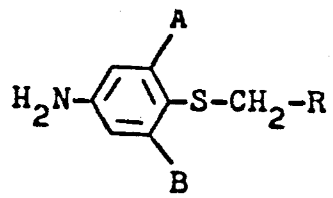

(II)

,

in der A, B und R die im Vorstehenden angegebene Bedeutung haben,
mit einer Verbindung der allgemeinen Formel (III)

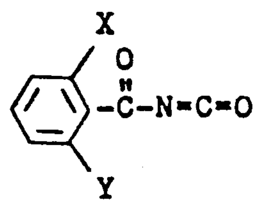

(III)

,

in der X und Y die im Vorstehenden angegebene Bedeutung haben, umgesetzt wird, oder

ii) eine Verbindung der allgemeinen Formel (IV)

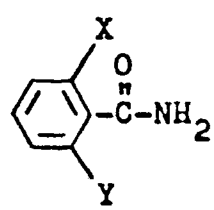

(IV)

,

in der X und Y die im Vorstehenden angegebene Bedeutung haben,
mit einer Verbindung der allgemeinen Formel (V)

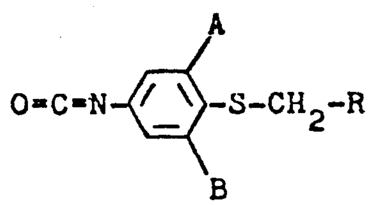

(V)

,

in der A, B und R die im Vorstehenden angegebene Bedeutung haben,
umgesetzt wird.

6. Verfahren zur Bekämpfung von Insekten, dadurch gekennzeichnet, daß auf Insekten oder den Lebensraum von Insekten eine insektizid wirksame Menge einer Verbindung nach Anspruch 1 bis 4 aufgebracht wird.

7. Insektizides Mittel, enthaltend als Wirkstoff ein Benzoylharnstoff-Derivat nach Anspruch 1 bis 4.

8. Neue Anilin-Derivate der allgemeinen Formel (II)

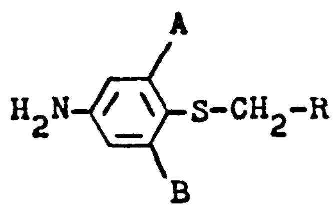

(II)

,

in der
A und B jeweils ein Wasserstoff-Atom, eine niedere Alkyl-Gruppe oder ein Halogen-Atom bezeichnen, mit der Maßgabe, daß A und B nicht gleichzeitig ein Wasserstoff-Atom bezeichnen, und
R eine fluoro-substituierte Alkyl-Gruppe mit 1 bis 2 Kohlenstoff-Atomen bezeichnet.

9. Verfahren zur Herstellung neuer Anilin-Derivate der allgemeinen Formel (II)

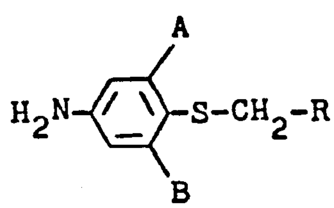

(II)

,

in der

A und B jeweils ein Wasserstoff-Atom, eine niedere Alkyl-Gruppe oder ein Halogen-Atom bezeichnen, mit der Maßgabe, daß A und B nicht gleichzeitig ein Wasserstoff-Atom bezeichnen, und

R eine fluoro-substituierte Alkyl-Gruppe mit 1 bis 2 Kohlenstoff-Atomen bezeichnet,

dadurch gekennzeichnet, daß eine Verbindung der allgemeinen Formel (VI)

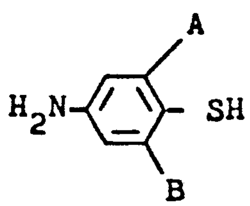

(VI)

,

in der A und B die im Vorstehenden angegebene Bedeutung haben,

mit einer Verbindung der allgemeinen Formel (VII)

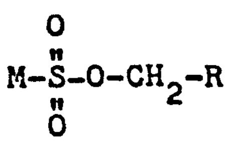

(VII)

,

in der R die im Vorstehenden angegebene Bedeutung hat und M eine niedere Alkyl-Gruppe oder eine Aryl-Gruppe bezeichnet, in Gegenwart einer Base umgesetzt wird.

10. Verfahren nach Anspruch 9, dadurch gekennzeichnet, daß ein aprotisches polares Lösungsmittel oder ein tertiärer Alkohol als Reaktionslösungsmittel verwendet wird.